# EUROPEAN PATENT SPECIFICATION

(11) **EP 2 150 195 B1**
(45) Date of publication and mention of the grant of the patent: **18.08.2010**
(21) Application number: 08760286.8
(22) Date of filing: 30.05.2008
(51) Int. Cl.: A61B 19/02, A61C 19/02, A61M 5/00, A61M 5/32, A61M 25/00

(54) **PACKING ALLOWING GAS STERILISATION**
FÜR GASSTERILISATION GEEIGNETE VERPACKUNG
CONDITIONNEMENT PERMETTANT UNE STÉRILISATION DE GAZ

(30) Priority: 06.06.2007 US 942326 P
(43) Date of publication of application: 10.02.2010
(73) Proprietor: Unomedical A/S, 3460 Birkerød (DK)
(72) Inventor: NIELSEN, Jens, Egebjerg, DK-4100 Ringsted (DK)
(74) Representative: Nilausen, Kim
(86) International application number: PCT/EP2008/056698
(87) International publication number: WO 2008/148714

(56) References cited:
- WO-A-2004/071308
- DE-A1- 4 342 329
- US-A- 3 306 291
- US-A- 4 863 016

## Description

This invention relates to a packing enclosing a medical device comprising a fabric or non-woven based layer e.g. provided with an adhesive surface or another layer for securing a device to the skin of a patient.

### Background of the invention

Sterilisation by gas is well known within sterilisation of medical equipment. E.g. from US patent no. 5.372.787 it is known to use a sterilizing container for medical and surgical instruments comprising a box with a bottom part and a cover for sealing the bottom part in a germ-tight manner, the side walls of the bottom part of the box have openings covered on the outside with germ-tight but sterilization gas permeable filter layers which are held in position by retaining plates mounted on the side walls, the cover of the box being locked to the bottom part by a locking structure formed at one end of the box at which the cover and bottom part includes retaining structures receiving a safety tag which covers the locking structure when the cover is closed in such a way that the safety tag is firmly positioned in front of the locking structure and the locking structure is only accessible by destroying the safety tag.

A packing enclosing a medical device is further known from the document DE 43 42 329 A1, from which the preamble of claim 1 emanates.

The present invention is a packing enclosing a medical device, e.g. an infusion set or another medical device provided with a skin penetrating member and intended for positioning on the patients skin, which medical device comprises a mounting pad or another filter layer as a part of the medical device i.e. the filter layer is unreleasably fastened to or is an unreleasable part of the medical device and will be removed together with the medical device when the medical device is removed from the packing. The filter layer or at least a part of the filter layer provides access for sterilisation gas and prevents access of germs. The packing is constructed in such a way that separate filter layers being part of the packing and not of the medical device to be sterilised are not needed.

### Summary of the invention

According to the present invention a packing enclosing a medical device is provided. This packing comprises at least one layer impenetrable to gas which layer has at least one opening, said opening is covered by a filter layer which is permeable for sterilization gas wherein the filter layer is a part of the enclosed medical device to be sterilised. This means that the filter layer is unreleasably secured to the medical device contrary to being unreleasably secured to the packing or being a separate part embedded in the packing.

Further the part of the filter layer surrounding the opening is placed in close contact with two opposing parts of the packing where a first part of the packing which is provided with the opening is in close contact with a first side of the filter layer and the second part of the packing is in close contact with the opposite side of the filter layer. In the embodiment shown in the figures the first part of the packing is constituted of a top part of the packing and the second part of the packing is constituted of a bottom part of the packing. The two parts are pressed together in order to create the close contact.

In one embodiment the filter layer is part of a mounting pad provided with a single sided adhesive coating and the adhesive coating can e.g. face the second part of the packing. Further the adhesive coating is provided with a release layer.

According to one embodiment the filter layer comprises a non-woven backing with good skin compatibility. The backing should be permeable to the used sterilisation gas which could e.g. be ethylene oxide gas.

According to one embodiment the packing is used for an infusion part comprising a body of a hard material, a mounting pad provided with an adhesive surface and a cannula.

### Description of the drawings

Embodiments of the invention will now be described with reference to the figures in which:
Figure 1 shows a cut through a first embodiment of a packing of an infusion set according to the invention.
Figure 2 shows a cut through a second embodiment of a packing of an infusion set according to the invention provided with a separate insertion needle.
Figure 3 shows a cut through the second embodiment of a packing of fig. 2 where the separate insertion needle is withdrawn from the cannula.
Figure 4 the embodiment of fig. 2 and 3 in a side view.

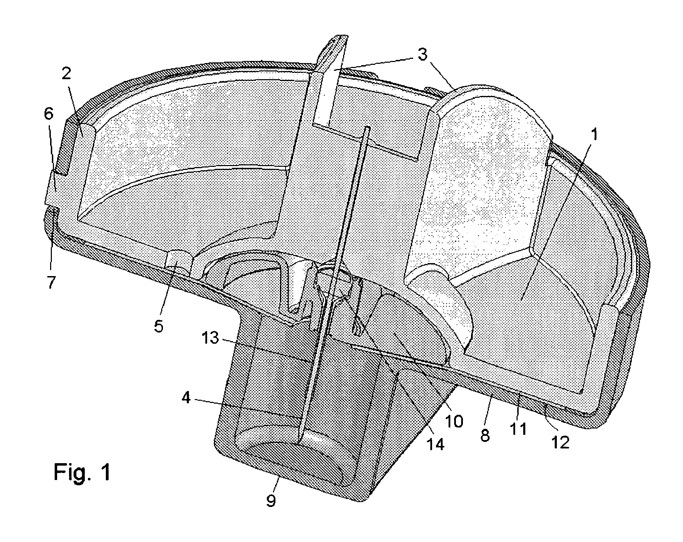

The packing of fig. 1 is constituted of two separate parts, a top part 1 and a bottom part 8, made of a relatively hard plastic. The top part 1 covers the side of the packed medical device which during use is turned away from the patient i.e. the distal side; the bottom part 8 covers the side of the packed medical device which during use is turned towards the patient i.e. the proximal side from which side a skin penetrating part extends. That the material is relatively hard means that the material keeps the form that it was given during making e.g. by molding.

In this embodiment the medical device is a port or gateway part which is comprising a body part 10, a mounting pad 11 having an adhesive surface 12, a cannula 13 and a septum 14 which is protecting the entrance of the cannula from being contaminated with microorganisms. The medical device could also be an infusion part provided with means for joining it with a delivering part delivering a defined amount of medication e.g. insulin, or the medical device could be a measuring device provided with a skin penetrating sensor.

The top part 1 comprises a cylindrical or upright circular wall 2 having at least one protruding part 6, a flat circular part which provides a close fit for at least a part of the mounting pad 11 and a central domed part which provides a close fit for the body 10 of the infusion part. The central domed part of this embodiment is provided with handle parts 3 which make it possible to separate the top part 1 from the bottom part 8.

In the embodiment shown in fig. 1 an introducer needle 4 is embedded in the top part 1, this means that the introducer needle 4 is unreleasably fastened to the top part 1 and is removed from the medical device when the top part 1 is separated from the medical device.

An opening 5 is placed in the flat circular part of the top part 1. The opening 5 is covered by the upper surface, i.e. the surface which is not adhesive, of the mounting pad 11. The upper surface of the mounting pad 11 is kept close to the edge of the opening 5 and thereby provides a germ-tight seal as the mounting pad 11 is squeezed against the top part 1 by the bottom part 8.

The opening 5 allows sterilization gas to enter into the inside of the packing and the close fit of the mounting pad 11 around the edge of the opening 5 prevents microorganisms from entering through the opening into the inside of the packing of the medical device.

The bottom part 8 comprises a cylindrical or upright circular wall having at least one open passage 7 corresponding to the at least one protruding part 6 of the top part 1, and a flat circular part corresponding to the flat circular part of the top part which together with the top part 1 provides a close fit for at least the part of the mounting pad 11 surrounding the opening 5. Further the bottom part 8 is provided with a central cylindrical part 9 providing a protected space for the introducer needle 4 and the cannula 13.

The top part 1 and the bottom part 8 are separated from each other by grapping the handle part 3 and rotating the top part 1 relative to the bottom part 8. When rotated the protruding part or parts 6 of the top part 1 will ensure that the top part 1 moves away from the bottom part 8 with a spiral motion.

The adhesive surface 12 of the mounting pad 11 is either provided with a separate protective layer covering the adhesive surface or the inner surface of the bottom part 8 can constitute such a protective layer. If the inner surface of the bottom part 8 constitutes the protective layer another motion than rotation would be suitable when separating the top part 1 and the medical device from the bottom part 8.

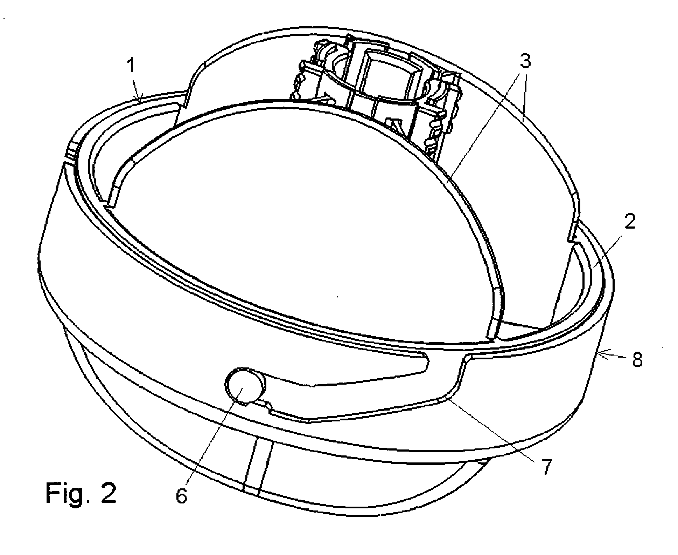

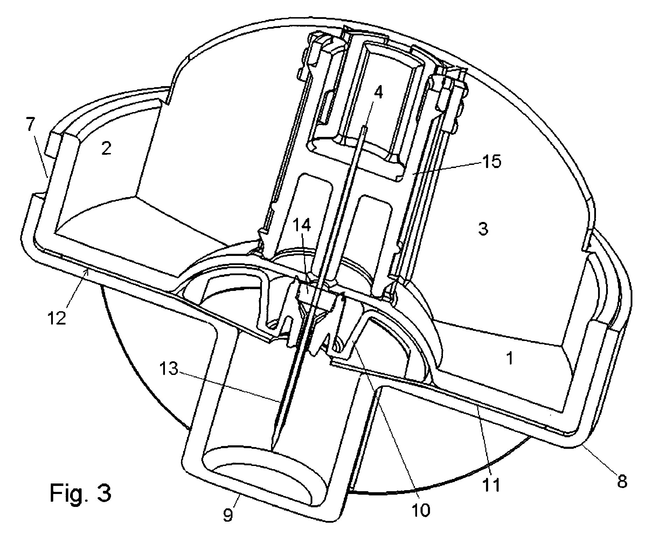

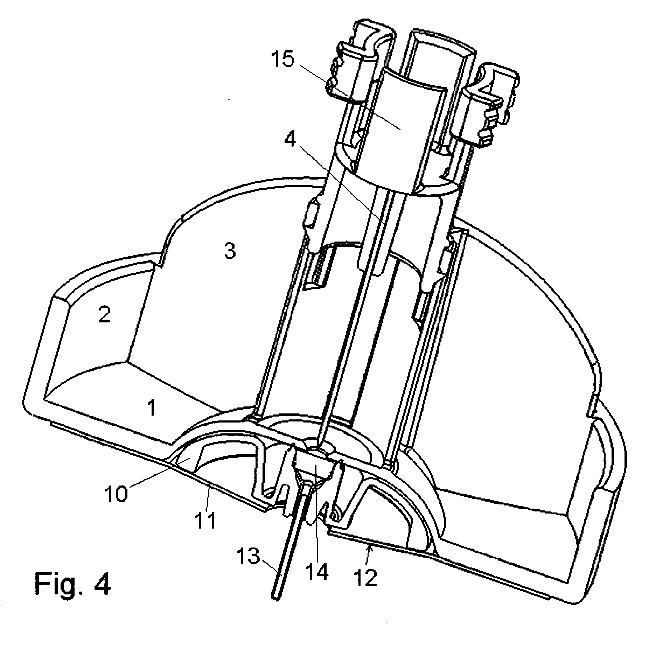

Figures 2, 3 and 4 show a second embodiment of a packing enclosing a medical device according to the invention. Same reference numbers are used for similar parts as shown in figure 1.

In the embodiment of fig. 2 the introducer needle 4 is embedded in a separate unit 15 which means that the unit 15 including the introducer needle 4 can be removed independently of the top part 1 from the medical device.

The mounting pad 11 should be made of a material which is permeable for a sterilization gas e.g. ethylene oxide, formaldehyde, ozone or another suitable kind of sterilization gas and at the same time the material of the mounting pad 11 should be impenetrable for microorganisms and germs.

An example of a suitable mounting pad material is a material constituted of a non-woven backing e.g. made of polyester, the backing is single sided coated with a pressure sensitive adhesive e.g. an acrylic adhesive. In one embodiment the pressure sensitive adhesive is covered with a release layer which layer protects the adhesive before use and assures an optimum performance of the adhesive layer. Such a release layer can be a silicone based paper.

Such a material is e.g. sold by *BSN medical* under the name "Klebevlies". This product has a proven biological safety according to MDD 93/42 which is equivalent to a CE marked BSN-medical product for wide area fixation (Fixomull stretch). "Klebevlies" consists of white, one-way stretchable polyester non-woven backing coated with a single sided with pressure sensitive acrylic adhesive. The adhesive is covered with silicone based release paper and the product is supplied in rolls. The adhesive is microporous which means that it is permeable to air and vapour and it has excellent skin compatibility.

The mounting pad e.g. can have the following physical properties (test method):

| | | |
|---|---|---|
| Adhesion to steel: | ≥ 1.0 N/cm | (JBS.40000083) |
| Air permeability: | ≥ 1.0 cm/cm/s | (JBS.40000050) |
| Coating weight of adhesive: | 47 g/m² ± 3 g/m² | (JBS.40000051) |
| Thickness of non-woven backing: | 0.48 ± 0.2 mm | |
| Thickness of release paper: | 77 ± 10 µm | |

## Claims

1. Packing enclosing a medical device (10, 11, 12, 13) which packing comprises at least one layer (1) impenetrable to gas which layer has at least one opening (5), said opening (5) is covered by a filter layer (11) which is permeable for sterilization gas, **characterized in that** the filter layer (11) is a part of the enclosed medical device to be sterilised.

2. Packing according to claim 1, **characterized in that** the part of the filter layer (11) surrounding the opening (5) is placed in close contact with two opposing parts of the packing where a first part of the packing (1) which is provided with the opening (5) is in close contact with a first side of the filter layer (11) and the second part of the packing (8) is in close contact with the opposite side of the filter layer (11).

3. Packing according to claim 2, **characterized in that** the filter layer (11) is part of a mounting pad provided with a single sided adhesive coating.

4. Packing according to claim 3, **characterized in that** the adhesive coating faces the second part of the packing (8).

5. Packing according to claim 3 or 4, **characterized in that** the adhesive coating is provided with a release layer.

6. Packing according to claim 3, 4 or 5, **characterized in that** the filter layer (11) comprises a non-woven backing with good skin compatibility.

7. Packing according to any preceding claim, **characterized in that** the medical device is an infusion part comprising a body (10) of a hard material, a mounting pad provided with an adhesive surface (12) and a cannula (13).

## Patentansprüche

1. Verpackung, welche eine medizinische Anordnung (10, 11, 12, 13) einschließt, welche Verpackung mindestens eine gasdurchlässige Schicht (1) umfasst, welche mindestens eine Öffnung (5) aufweist, welche Öffnung (5) durch eine für ein Sterilisationsgas durchlässige Filterschicht (11) überdeckt ist, **dadurch gekennzeichnet, dass** die Filterschicht (11) einen Teil der eingeschlossen, zu sterilisierenden medizinischen Anordnung bildet.

2. Verpackung nach Anspruch 1, **dadurch gekennzeichnet, dass** der die Öffnung (5) umgebende Teil der Filterschicht (11) in nahem Kontakt mit zwei gegenüberliegenden Teilen der Verpackung angeordnet ist, wobei ein mit der Öffnung (5) vorgesehener erster Teil der Verpackung (1) sich in nahem Kontakt mit einer ersten Seite der Filterschicht (11) befindet, und der zweite Teil der Verpackung (8) sich in nahem Kontakt mit der gegenüberliegenden Seite der Filterschicht (11) befindet.

3. Verpackung nach Anspruch 2, **dadurch gekennzeichnet, dass** die Filterschicht (11) Teil einer mit einer einseitigen Klebebeschichtung versehenen Befestigungsmatte ist.

4. Verpackung nach Anspruch 3, **dadurch gekennzeichnet, dass** die Klebebeschichtung nach dem zweiten Teil der Verpackung (8) gewandt ist.

5. Verpackung nach Anspruch 3 oder 4, **dadurch gekennzeichnet, dass** die Klebebeschichtung mit einer Freisetzungsschicht versehen ist.

6. Verpackung nach Anspruch 3, 4 oder 5, **dadurch gekennzeichnet, dass** die Filterschicht (11) eine Vliesrückseite mit einer guten Hautverträglichkeit umfasst.

7. Verpackung nach einem der vorgehenden Ansprüche, **dadurch gekennzeichnet, dass** die medizinische Anordnung ein Infusionsteil ist, welcher einen Körper (10) aus einem harten Werkstoff, eine mit einer Klebefläche (12) versehene Befestigungsmatte und eine Kanüle (13) umfasst.

## Revendications

1. Conditionnement englobant un dispositif médical (10, 11, 12, 13), ledit conditionnement comprenant au moins une couche (1) impénétrable aux gaz, ladite couche ayant au moins une ouverture (5), ladite ouverture (5) étant couverte d'une couche filtre (11) qui est perméable aux gaz de stérilisation, **caractérisé en ce que** la couche filtre (11) fait partie du dispositif médical à être stérilisé.

2. Conditionnement selon la revendication 1, **caractérisé en ce que** la partie de la couche filtre (11) encadrant l'ouverture (5) est positionnée en contact étroit de deux éléments opposants du conditionnement, dans lequel un premier élément du conditionnement (1) qui est pourvu de l'ouverture (5) est en contact étroit avec une première coté de la couche filtre (11) et le deuxième élément du conditionnement (8) est en contact étroit avec la coté opposée de la couche filtre (11).

3. Conditionnement selon la revendication 2, **caractérisé en ce que** la couche filtre (11) fait partie d'une rondelle de montage pourvue d'un revêtement adhésif simple.

4. Conditionnement selon la revendication 3, **caractérisé en ce que** le revêtement adhésif se dirige vers le deuxième élément du conditionnement (8).

5. Conditionnement selon la revendication 3 ou 4, **caractérisé en ce que** le revêtement adhésif est pourvu d'un revêtement de dégagement.

6. Conditionnement selon les revendications 3, 4 ou 5, **caractérisé en ce que** la couche filtre (11) comprend un support non-tissé ayant bonne compatibilité avec la peau.

7. Conditionnement selon l'une quelconque des revendications précédentes, **caractérisé en ce que** le dispositif médical est un élément de perfusion comprenant un corps (10) d'un matériau dur, une rondelle de montage ayant une surface adhésive (12) et une canule (13).
